# EUROPÄISCHE PATENTSCHRIFT

(11) **EP 0 720 474 B1**
(45) Veröffentlichungstag und Bekanntmachung des Hinweises auf die Patenterteilung: **21.07.1999**
(21) Anmeldenummer: 94928368.3
(22) Anmeldetag: 16.09.1994
(51) Int. Cl.: A61K 9/70

(54) **LÖSEMITTELFREI HERSTELLBARES WIRKSTOFFPFLASTER ENTHALTEND FLÜCHTIGE INHALTSSTOFFE**
VOLATILE ACTIVE SUBSTANCE CONTAINING PLASTER THAT MAY BE PRODUCED WITHOUT SOLVENTS
PANSEMENT CONTENANT DES PRINCIPES ACTIFS VOLATILS SUSCEPTIBLE D'ETRE PRODUIT SANS SOLVANTS

(30) Priorität: 22.09.1993 DE 4332094
(43) Veröffentlichungstag der Anmeldung: 10.07.1996
(73) Patentinhaber: LTS LOHMANN Therapie-Systeme GmbH, 56567 Neuwied (DE)
(72) Erfinder: HORSTMANN, Michael, D-56564 Neuwied (DE)
(74) Vertreter: Flaccus, Rolf-Dieter, Dr.
(86) Internationale Anmeldenummer: EP9403107
(87) Internationale Veröffentlichungsnummer: WO9508324

(56) Entgegenhaltungen:
- EP-A- 0 227 836
- EP-A- 0 305 756
- EP-A- 0 318 385
- EP-A- 0 413 034
- WO-A-88/10111
- DE-A- 3 843 238
- DATABASE WPI Section Ch, Week 9326, Derwent Publications Ltd., London, GB; Class A04, AN 93-208822 & JP,A,5 132 418 (LINTEC CORP.) 28. Mai 1993 & PATENT ABSTRACTS OF JAPAN vol. 17, no. 496 (C-1108) 8. September 1993 & JP,A,05 132 418 (LINTEC CORP) 28. Mai 1993 & CHEMICAL ABSTRACTS, vol. 119, no. 14, 4. Oktober 1993, Columbus, Ohio, US; abstract no. 146627, OKABE HIDEAKI ET AL 'MANUFACTURE OF MEDICAL ADHESIVE SUPPORTS IMPREGNATED WITH VOLATILE SUBSTANCES'

## Beschreibung

Die Erfindung betrifft ein wirkstoffhaltiges Pflaster zur Abgabe von Wirkstoffen über die Haut an den menschlichen Körper.

Wirkstoffhaltige Pflaster sind auch unter der speziellen Bezeichnung transdermale therapeutische Systeme (TTS) in der arzneilichen Therapie einer Reihe von Erkrankungen bereits im Markt eingeführt.

Viele der für die transdermale Therapie besonders geeigneten Wirkstoffe sind bereits bei Raumtemperatur, zumindest aber bei üblichen Verarbeitungstemperaturen von 60 bis 80°C, merklich flüchtig. Dies ist störend, weil sich durch das schwer voraussehbare Ausmaß des jeweiligen Wirkstoffverlustes im Prozeß keine sichere Dosierung des Arzneimittels erreichen läßt.
Flüchtigkeit im Sinne der vorliegenden Erfindung liegt in jedem Fall dann vor, wenn ein flaches Gefäß, gefüllt mit einem bodenbedeckenden Überschuß der Reinsubstanz bei 100°C, kontinuierlich pro Stunde mindestens 0,1 mg Stoff auf 10 cm² freier Oberfläche an die Umgebung abgibt. In besonderen Fällen, wie bei hochwirksamen pharmazeutischen Wirkstoffen, deren Dosierung in TTS typisch bei Flächenkonzentrationen um 1 mg auf 10 cm² oder weniger liegt, kann bereits eine geringe Flüchtigkeit störend sein. Aus diesem Grunde gibt die vorstehende Definition nur eine grobe Richtschnur.

Die Problematik der Flüchtigkeit betrifft auch leicht flüchtige Inhaltsstoffe in TTS, die keine eigenen therapeutischen Wirkungen entfalten, aber die Aufgabe haben, den Wirkstofffluß durch die Haut zu steigern. Für diesen Zweck eignen sich beispielsweise:

Benzylalkohol, Butanol und andere kurzkettige Alkohole, Triglyceride, hochsiedende aliphatische Kohlenwasserstoffe, Glycerin, Glycerinmonooleat, Isopropylmyristat oder andere kurzkettige Ester, Menthol oder andere flüchtige Terpenderivate (die Gemischbestandteile vieler natürlicher etherischer Öle sind), Octanol-1 und andere flüchtige mittelkettige Alkohole (z.B. auch Oleylalkohol), Octansäure und andere mittelkettige aliphatische Carbonsäuren und viele weitere Stoffe.

Die gemeinsame herstellungstechnische Problematik flüchtiger Wirk- und Hilfsstoffe (im folgenden zusammenfassend als "leicht flüchtige Inhaltsstoffe" bezeichnet) hat zu einer Vielzahl von Vorschlägen meist relativ aufwendiger Konstruktionen für TTS, die solche Zusatzstoffe enthalten, geführt.
So wird in einem beutelartigen Reservoir eine größere Menge Ethanol zusammen mit dem (hier nicht flüchtigen) Wirkstoff vorrätig gehalten; während der Anwendung passieren Hilfsstoff wie Wirkstoff eine Kontrollmembran (US-Patent 4,379,454).

Da für den Anwender ein dünner, flexibler Aufbau wichtig ist, war es wünschenswert, den Zusatz leicht flüchtiger Zusatzstoffe auch in einfacheren Pflastern - im Idealfall nur aus einer selbstklebenden Matrixschicht und einer nichtklebenden Rückschicht bestehend - zu realisieren. Dies wurde auch häufig vorgeschlagen, zum Beispiel werden in DE-OS 42 10 165 einfache Matrix-TTS mit Zusatz von polaren und unpolaren Penetrationsverstärkern beschrieben.

Nach dieser klassischen Technik werden Matrix-TTS auch mit Anteilen solcher leichtflüchtigen Inhaltsstoffe, die in der Arzneiform verbleiben sollen, so hergestellt, daß man eine Lösung des Klebers in einem niedrigsiedenden Lösungsmittel mit dem Wirkstoff und dem flüchtigen Inhaltsstoff mischt, die Mischung folienartig auf einer Basisfolie aufträgt, das flüchtige Lösungsmittel durch Erwärmen (meist bis auf 50 bis 80°C) entfernt und das erhaltene Produkt mit einer abziehbaren Schutzfolie abdeckt.
Dabei werden zur Auflösung des Klebers geeignete Lösungsmittel - daß heißt solche, die noch leichter flüchtig sind als die zum Verbleib im System zugesetzten Inhaltsstoffe - wie Methanol, Ethanol oder Isopropanol, Benzin, Aceton, Ethylacetat, etc. verwendet.

Viele der in DE-OS 42 10 165 genannten Zusatzstoffe, zum Beispiel Pinen, Limonen oder Myrcen sind jedoch unter diesen Bedingungen so stark flüchtig, daß eine reproduzierbare Dosiermöglichkeit mit dem beschriebenen einschichtigen Systemaufbau und dem geschilderten Verfahren nicht mehr möglich ist.

Bei einem alternativen Herstellungsverfahren, dem Heißschmelzverfahren, wie es in DE 37 43 946 für die transdermale Anwendung des flüchtigen Wirkstoffes Nitroglycerin beschrieben ist, ergeben sich derartige Probleme zwar weniger, weil Wirkstoff und Hilfsstoffe in einem geschlossenen System miteinander aufgeschmolzen werden und nur kurzzeitig nach der Beschichtung mit der Außenluft in Verbindung kommen. Andererseits sind eine Reihe von Hilfe- und Wirkstoffen nicht für dieses Verfahren geeignet, weil die Hilfestoffe nicht genügend thermoplastisch sind, die Wirkstoffe zu temperaturempfindlich oder weil der Zusatz an plastifizierenden, aber flüchtigen Hilfsstoffen zu gering ist, um ausreichend niedrige Verarbeitungstemperaturen zu gewährleisten.

Die Möglichkeit des Einbringens eines mit flüchtigem Wirkstoff, z.B. Nicotin, bedruckten Vliesstückes löst zwar das Verdampfungsproblem, führt aber zu vergleichsweise dicken Arzneistoffpflastern.

In DE 32 31 400 wird von der Migration von Wirkstoffen oder Adjuvantien zwischen Matrixschichten Gebrauch gemacht, um Rekristallisationen von Wirkstoff und Adjuvans zu vermeiden. Es werden hier jedoch lediglich jeweils mit Hilfe- oder Wirkstoff angereicherte Matrixschichten beschrieben, die nach Laminierung und Migration zu einem TTS mit vorteilhaftem Wirkstofffluß führen. Eine Verwendung des Wirkstoffes oder der Adjuvantien selbst als bei Raumtemperatur nutzbarem Lösemittel wird dort nicht erwähnt. Entsprechend wurden auch die Vorteile des Migrationsprozesses zur Erzielung scherfester TTS mit flüchtigen Inhaltsstoffen dort nicht erkannt.

In EP 0 249 475 wird ein Abgabesystem beschrieben, welches aus unmittelbar vor Gebrauch zu vereinigenden Anteilen besteht. Nach Vereinigung wandert Wirkstoff aus der wirkstoffhaltigen Schicht in die zunächst völlig wirkstofffreie Schicht, wobei eine beabsichtigte Verzögerung der Abgabe erreicht wird.In diesem System ist zum Zeitpunkt der therapeutischen Anwendung noch keine Migration des Wirkstoffes erfolgt. Das dort beschriebene System zeigt also nicht die therapeutischen und anwendungstechnischen Vorteile eines Systems auf, bei dem zum Zeitpunkt der Anwendung bereits eine Migration erfolgt ist. Die im System nach EP 0 249 475 beschriebene Verzögerung der Wirkstoffabgabe ist in den meisten Fällen unerwünscht. Das System nach EP 0 249 475 ist auch insofern nachteilig, als es keine Lehre enthält, wie mit leichtflüchtigen Wirkstoffen zu verfahren ist und dementsprechend die genaue Dosierung solcher Stoffe in transdermalen Systemen nicht beschreibt.

Das Konzept, die Migration von Inhaltsstoffen ganz allgemein zu nutzen, ist bekannt.
In DE-PS 36 29 304 wird die Dosierung von flüchtigen Wirkstoffen auf ein in der Fläche kleineres, absorptives Substrat beschrieben, die anschließend - vor der Anwendung - sich durch Migration in den angrenzenden Matrixschichten verteilen.

Eine Abwandlung dieser Technik, angewandt auf vollfächige Laminate findet sich in US 4,915,950. Hier wird der Inhaltsstoff, es kann durchaus auch eine flüchtige Substanz, z.B. ein etherisches Öl sein - auf ein poröses, absorbierendes Substrat durch Bedrucken aufgetragen, von der er sich anschließend -vor der Anwendung - durch Migration in die angrenzenden Matrixschichten verteilt. Dieses System zeigt zwar einige Vorteile, zum Beispiel die Vermeidung der Verdampfung von Wirkstoff bei der Produktion, die arbeitsschutzrelevante Konsequenzen haben kann. Allerdings ist der Aufbau resultierender TTS noch recht kompliziert und eine textile oder auf andere Art poröse Zwischenschicht bedeutet eine unerwünschte Starrheit mit der möglichen Konsequenz verschlechterter Verklebung mit der Haut.

Aufgabe der vorliegenden Erfindung ist es daher, ein wirkstoffhaltiges Pflaster bereitzustellen, welches die nahezu verlustfreie Einarbeitung von bei der üblichen Verarbeitungstemperatur flüchtigen Wirk- oder Hilfsstoffen ermöglicht, einfachen Aufbau aufweist und über verbesserte Trageeigenschaften auf der Haut verfügt.

Diese Aufgabe wird erfindungsgemäß gelöst durch ein wirkstoffhaltiges Pflaster mit geschichtetem Aufbau, bestehend aus einer im wesentlichen wirkstoffundurchlässigen Rückschicht, mindestens zwei wirkstoffhaltigen Matrixschichten und einer wiederablösbaren Schutzschicht, wobei die den leicht flüchtigen Wirkstoff enthaltende Matrixschicht auf einen separat hergestellten Verbund aus einer oder mehreren weiteren Matrixschichten auflaminiert ist, gekennzeichnet dadurch daß die erste Matrixschicht bei der Herstellung eine streichfähige molekulardisperse Lösung des Matrixgrundmaterials in dem leicht flüchtigen Inhaltsstoff als ausschließlichem Lösungsmittel darstellt und daß der nach dem Laminieren erhaltene Verbund anschließend nach Migration des leicht flüchtigen Inhaltsstoffes ausreichend scherstabil wird, wie es zur Verwendung als Wirkstoffpflaster erforderlich ist.

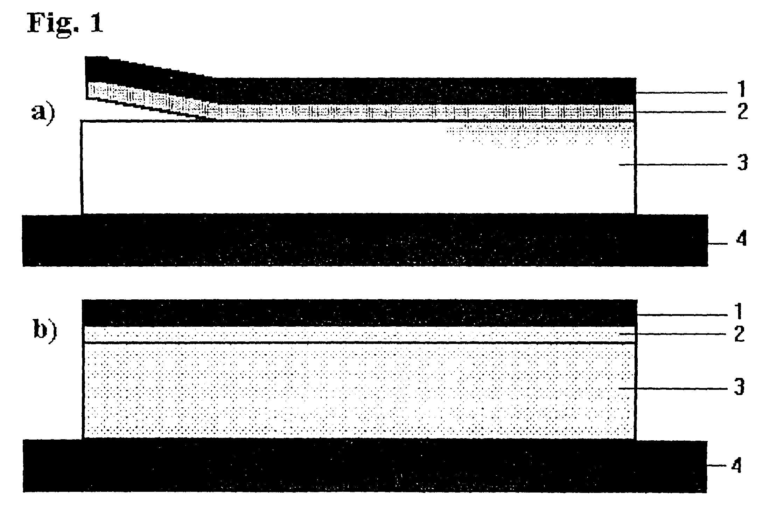

Grundgedanke der Erfindung ist einerseits die Aufteilung der Matrixschicht in zwei aufeinander zu laminierende Anteile, von denen der eine Teil aus Matrixschicht mit leicht flüchtigem Inhaltsstoff und einer wirkstoffundurchlässigen Folie besteht. Dabei ist kennzeichnend, daß diese "erste Matrixschicht" (2 in Fig. 1) während des Herstellungsprozesses eine streichfähige, molekulardisperse Lösung des Matrixgrundmaterials in dem leicht flüchtigen Inhaltsstoff als ausschließlichem Lösungsmittel darstellt. Da die übrigen, für den leicht flüchtigen Inhaltsstoff durch Migration zugänglichen Bereiche des TTS (3 in Fig.1) sehr aufnahmefähig für den leicht flüchtigen Inhaltsstoff ausgeführt sind und darüber hinaus grundsätzlich dicker sind als die erste Matrixschicht, verliert sich kurz nach der Herstellung auch die streichfähig-dickflüssige Konsistenz dieser Schicht - das Gesamtsystem wird weich-klebrig, aber insgesamt formstabil.

Während die übrigen Teile des TTS konventionell durch Lösemitteltrocknung, durch Heißschmelztechniken oder aber auch durch die Beschichtung mit Kleberdispersionen in Wasser hergestellt werden können, werden zur Herstellung der "ersten Matrixschicht" die zumeist polymeren Hilfsstoffe in einem höheren Anteil des leicht flüchtigen Inhaltsstoffes gelöst und auf eine der beiden Prozeßfolien (wiederablösbare Schutzfolie oder endgültige Rückschicht) durch genau kontrollierte Auftragstechniken aufgebracht. Hierbei sind Dünnfilmtechniken, u.a. auch Drahtrakelauftrag, besonders zu erwähnen. Wichtig ist, daß dieser Matrixschicht keine längere offene Lagerung gestattet wird, da sonst, wenn auch in geringem Umfang - Verdunstung droht. Am besten findet unmittelbar anschließend an das Beschichten Laminieren auf den in der Regel dickeren, bereits vorgefertigten Rest der Matrix statt. Danach findet Migration der leichtflüchtigen Komponente statt, so daß der angereicherte Bereich seine extreme Plastizität verliert und die Matrix insgesamt eine den therapeutischen Erfordernissen entsprechende Scherfestigkeit erhält. Die Dauer dieses Vorgangs ist neben anderen physikalischen Parametern abhängig von den Diffusionseigenschaften aller Inhaltsstoffe sowie von der Gesamtgeometrie; üblicherweise ist er nach wenigen Minuten bis einigen Stunden beendet. In einzelnen Fällen kann aber auch eine Reifungszeit von einigen Tagen oder sogar einer Woche in Kauf genommen werden. Dies ist bei sehr dicken TTS (über ca. 1000 µm Matrixgesamtdicke) bei schwach diffusiblen flüchtigen Inhaltsstoffen und niedriger Umgebungstemperatur möglich.

Da das Gesamtsystem nach der Laminierung beidseitig durch undurchlässige Folien geschützt ist, kann dieser Wanderungs- oder Reifungsprozeß durch eine kurzieitige Wärmebehandlung intensiviert und beschleunigt werden. Übliche Bedingungen sind hier bei der Einwirkung von Temperaturen um 30 bis 60°C innerhalb von 20 Sekunden bis 30 Minuten gegeben.
Durch die Aufteilung der Matrix in zwei Bereiche, von denen nur einer die leichtflüchtige Komponente führt, kann der Bereich, welcher frei von diesen Stoffen hergestellt wird, zunächst konventionell gefertigt und bereitgestellt werden.

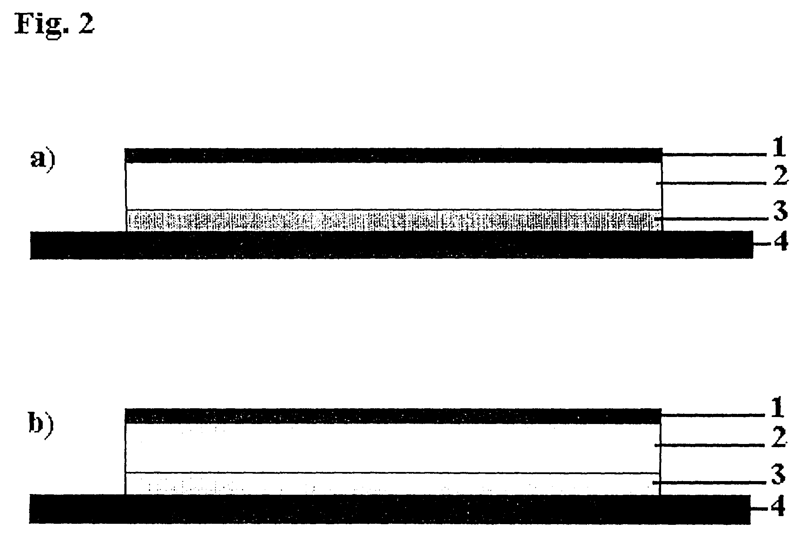

Es ist grundsätzlich möglich, die zunächst den leichtflüchtigen Inhaltsstoff enthaltende Schicht zur Haut hin (vor der Anwendung zur wiederablösbaren Schutzfolie hin) anzuordnen, wie in Fig. 2 dargestellt, aber es ist genausogut möglich, diese Schicht anzuordnen, wie in Fig. 1 dargestellt.

Weist der leichtflüchtige Inhaltsstoff gleiche Löslichkeit in allen Matrixschichten auf, so findet man nach Migration in allen Matrixschichten praktisch gleiche Konzentrationen dieses Inhaltsstoffes vor (Fig. 1 und 2). Dies ist insbesondere dann der Fall, wenn die Zusammensetzung der nichtflüchtigen Inhaltsstoffe in allen verwendeten Schichten gleich ist. Es ist aber durchaus auch möglich, durch entsprechende Wahl der Löslichkeiten in den eizelnen Matrix-schichten eine unterschiedliche Endkonzentration an leichtflüchtigem Inhaltsstoff in jeder einzelnen Matrixschicht zu erreichen, sofern dies gewünscht wird (Fig. 3).

Eine mögliche Anwendung findet das erfindungsgemäße Aufbauprinzip beispielsweise bei einer Form eines Acetylsalicylsäure-Pflasters, welches unter Zufuhr des relativ leicht flüchtigen Zusatzstoffes Limonen besonders hohe Hautpermeabilität gewinnt. Mit Limonen lassen sich zahlreiche polymere Grundmaterialien in eine streichfähige Konsistenz bringen.

Beispiele für leicht flüchtige Inhaltsstoffe sind z.B. die Hilfsstoffe 2-Pyrrolidon, Benzylalkohol, Butanol und andere kurzkettige Alkohole, Cineol, Diethylenglycol, Diethylenglycolmonoethylether, Diisopropyladipat, Dimethyldecylphosphoxid, Dimethylisosorbid, Dimethyllauroylamid, Polydimethylsiloxan, Dimethylsulfoxid, Dodecylsulfoxid, Essigsäure, Ethylacetat und andere flüchtige aliphatische und aromatische Ester (die Gemischbestandteile vieler etherischer Öle sind), Ethylenglycol, Ethylenglycolmonolaurat und andere Ester und Ether von Ethylenglycol oder Propylenglycol, 2-Octyldodecanol, Glycerin, Glycerinmonooleat, Glycerinmonostearat, hydriertes Rizinusöl, Isopropylmyristat, Isopropylpalmitat, Menthol oder andere flüchtige Terpenderivate (die Gemischbestandteile vieler natürlicher etherischer Öle sind), Methylbenzoat, Methyloctylsulfoxid, Mono- oder Diethylacetamid, N,N-Diethyl-m-toluamid, N-Methylpyrrolidon, Octanol-1 und andere flüchtige mittelkettige Alkohole, Octansäure und andere mittelkettige aliphatische Carbonsäuren, Oleylalkohol, Olivenöl, Ölsäure, Ölsäureoleylester, Phenylethanol, Propylenglycol, Rizinolsäure, Triacetin, aber auch Mischungen solcher Stoffe wie zum Beispiel Ölsäure/Propylenglycol oder Limonen/Dimethylisosorbid.

Beispiele für leicht flüchtige pharmakologische Wirkstoffe sind Nicotin, Nitroglycerin, Salicylsäure, Scopolamin, Benzatropin, Fenfluramin, Cyclopentamin, Ephedrin und viele andere mehr.

Das Grundmaterial der ersten Matrixschicht kann identisch mit dem der übrigen Schichten sein, sich aber auch deutlich unterscheiden. Kritisch ist lediglich, daß die dem Fachmann eigene Sorgfalt bei der Auswahl der Basismaterialien geübt wird, um eine Laminierbarkeit zu erreichen.

Für alle Matrixschichten des erfindungsgemäßen Pflasters kommen daher Acrylsäureester enthaltende Copolymere, Mischungen aus Kautschuken und Harzen, Polyvinylacetat, Siliconpolymere und viele andere auf der Haut unbedenkliche Grundmaterialien in Frage. Der Zusatz von bis zu 40% Füllstoffen wie Titandioxid, Zinkoxid, Kreide, Aktivkohle, feinverteiltes Siliciumdioxid etc. behindert keineswegs die erfindungsgemäße Funktion und kann Vorteile hinsichtlich der Kohäsion der gefertigten Systeme besitzen.

Die Erfindung wird beispielhaft durch die Fig. 1 bis 3 und die Ausführungsbeispiele erläutert:

Die Fig. 1 bis 3 zeigen jeweils ein TTS mit
1. Rückschicht,
2. einer ersten Matrixschicht,
3. einer zweiten Matrixschicht,
4. ablösbarer Schutzschicht im Zustand
   a) vor Migration
   b) nach Migration

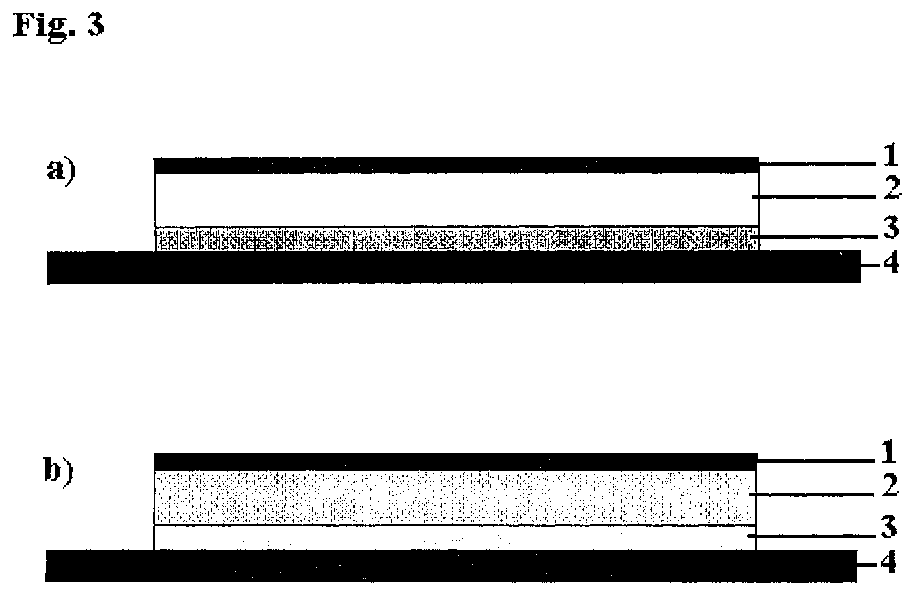

In einzelnen zeigen:
- Fig. 1:: Schicht mit leichtflüchtigem Inhaltsstoff zur Rückschicht hin;
gleiche Endkonzentration in den Matrixschichten nach Migration
- Fig. 2:: Schicht mit leicht flüchtigem Inhaltsstoff zur ablösbaren Schutzschicht hin;
gleiche Endkonzentration in den Matrixschichten nach Migration
- Fig. 3:: Schicht mit leichtflüchtigem Inhaltsstoff zur ablösbaren Schutzschicht hin;
unterschiedliche Endkonzentration an leichtflüchtigem Inhaltsstoff in jeder Matrixschicht nach Migration

### Beispiel 1:

100 g lösemittelfreies Acrylatcopolymer (Durotak 280-1753) und 1 g Estradiol werden in 300 ml Pfefferminzöl unter Rühren gelöst und in einer Schichtdicke von 30 µm auf eine 15 µm dicke Polyesterfolie beschichtet. Sogleich anschließend wird ein Verbund aus siliconisierter 75 µm dicker Polyesterfolie, beschichtet mit 200 g/m² Acrylatcopolymer (Durotak 280-1753) auf der jeweils klebrigen Seite hinzulaminiert. Nach 30 Minuten Lagerung bei Raumtemperatur weist das fertige Laminat nach Entfernen der Schutzfolie exzellente Klebeigenschaften auf ohne zu schmieren.

### Beispiel 2:

30 g Styrol-Isopren-Copolymer, 20 g Methylester des hydrierten Kolophoniums und 50 g Glycerinester des hydrierten Kolophoniums werden in 120 ml reinem Nicotin unter Rühren gelöst und in einer Schichtdicke von 40 µm auf eine 21 µm dicke Polyesterfolie beschichtet. Sogleich anschließend wird ein Verbund aus siliconisierter 100 µm dicker Polyesterfolie, in der Hitze beschichtet mit 300 g/m² Gemisch aus Styrol-Isopren-Copolymer/Methylester des hydrierten Kolophoniums/Glycerinester des hydrierten Kolophoniums auf der jeweils klebrigen Seite hinzulaminiert. Nach 60 Minuten Lagerung bei 40°C weist das fertige Laminat nach Entfernen der Schutzfolie exzellente Klebeigenschaften auf ohne zu schmieren.

## Patentansprüche

1. Wirkstoffhaltiges Pflaster mit haftklebender Fixiereinrichtung, enthaltend mindestens einen leicht flüchtigen Inhaltsstoff, bestehend aus einer im wesentlichen wirkstoffundurchlässigen Rückschicht, mindestens zwei wirkstoffhaltigen Matrixschichten und einer wiederablösbaren Schutz-schicht, wobei die den leicht flüchtigen Wirkstoff enthaltende Matrixschicht auf einen separat hergestellten Verbund aus einer oder mehreren wirkstofffreien weiteren Matrixschichten auflaminiert ist, dadurch gekennzeichnet, daß die erste wirkstoffhaltige Matrixschicht aus einer streichfähigen molekulardispersen Lösung des Matrixgrundmaterials in dem leicht flüchtigen Inhaltsstoff als ausschließlichem Lösungsmittel besteht, wobei der nach dem Laminieren erhaltene Verbund nach Migration des leicht flüchtigen Inhaltsstoffes in den Verbund eine insgesamt scherstabile Wirkstoffmatrix bildet.

2. Wirkstoffhaltiges Pflaster nach Anspruch 1, dadurch gekennzeichnet, daß der leicht flüchtige Inhaltsstoff ein pharmazeutisch aktiver Wirkstoff ist.

3. Wirkstoffhaltiges Pflaster nach Anspruch 1, dadurch gekennzeichnet, daß der leicht flüchtige Inhaltsstoff die Permeation in die Haut fördernde Eigenschaften aufweist.

4. Wirkstoffhaltiges Pflaster nach einem oder mehreren der voranstehenen Ansprüche, dadurch gekennzeichnet, daß die der Haut zugewandte Schicht haftklebende Eigenschaften aufweist.

5. Wirkstoffhaltiges Pflaster nach einem oder mehreren der voranstehenden Ansprüche, dadurch gekennzeichnet, daß die erste Matrixschicht bei der Herstellung mindestens 40 Gew.-% des leicht flüchtigen Inhaltsstoffes enthält.

6. Wirkstoffhaltiges Pflaster nach einem oder mehreren der voranstehenden Ansprüche, dadurch gekennzeichnet, daß alle Matrixschichten identische Zusammensetzung bezüglich aller nicht flüchtigen Inhaltsstoffe aufweisen und nach beendeter Migration des leicht flüchtigen Inhaltsstoffes eine in der Zusammensetzung einheitliche, monolithische Matrix entsteht.

7. Wirkstoffhaltiges Pflaster nach einem oder mehreren der voranstehenden Ansprüche, dadurch gekennzeichnet, daß die erste Matrixschicht, welche den leicht flüchtigen Inhaltsstoff enthält, bei der Herstellung eine geringere Dicke als die übrigen Matrixschichten aufweist.

8. Wirkstoffhaltiges Pflaster nach einem oder mehreren der vorangehenden Ansprüche, dadurch gekennzeichnet, daß die erste Matrixschicht, welche den leicht flüchtigen Inhaltsstoff enthält, bei der Herstellung eine Schichtdicke zwischen 2 und 100 µm, vorzugsweise 10 bis 30 µm aufweist.

9. Wirkstoffhaltiges Pflaster nach einem oder mehreren der voranstehenden Ansprüche, dadurch gekennzeichnet, daß der leicht flüchtige Inhaltsstoff ein Gemisch von leicht flüchtigen Substanzen ist.

10. Wirkstoffhaltiges Pflaster nach einem oder mehreren der vorangehenden Ansprüche, dadurch gekennzeichnet, daß der enthaltene Wirkstoff Acetylsalicylsäure oder eines ihrer pharmazeutisch unbedenklichen Salze ist.

11. Wirkstoffhaltiges Pflaster nach Anspruch 9 oder 10, dadurch gekennzeichnet, daß der Hauptbestandteil des genannten Gemisches leicht flüchtiger Substanzen Limonen ist.

12. Verfahren zur Herstellung wirkstoffhaltiger Pflaster gemäß einem oder mehreren der voranstehenden Ansprüche, dadurch gekennzeichnet, daß die den leicht flüchtigen Inhaltsstoff enthaltende Schicht gleichmäßig auf die wiederablösbare Schutzschicht aufgetragen wird und daß nach Auflaminierung eines Laminats, bestehend aus einer zweiten Matrixschicht und der Rückschicht nach beendeter Migration, ein pharmazeutisch wirksames Pflaster erhalten wird.

13. Verfahren zur Herstellung wirkstoffhaltiger Pflaster nach Anspruch 12, dadurch gekennzeichnet, daß der nach dem Laminieren erhaltene Verbund einer Wärmebehandlung zur Beschleunigung der Migration unterworfen wird.

## Claims

1. Active substance-containing plaster with a pressure sensitive fixing device, containing at least one readily volatile ingredient, consisting of a substantially active substance-impermeable backing layer, at least two active substance-containing matrix layers and a removable protective layer, whereby the matrix layer containing the readily volatile active substance is laminated on a separately produced composite of one or more further matrix layers which are active substance-free, characterized in that the first active substance-containing matrix layer consists of a spreadable molecularly disperse solution of the matrix base material in the readily volatile ingredient as the exclusive solvent, the composite which is obtained after laminating forming an active substance matrix that is as a whole shear-resistant after migration of the readily volatile ingredient into the composite.

2. Active substance-containing plaster according to claim 1, characterized in that the readily volatile ingredient is a pharmaceutically active agent.

3. Active substance-containing plaster according to claim 1, characterized in that the readily volatile ingredient has properties enhancing the permeation into the skin.

4. Active substance-containing plaster according to one or more of the preceding claims, characterized in that the layer facing the skin has pressure-sensitive adhesive properties.

5. Active substance-containing plaster according to one or more of the preceding claims, characterized in that the first matrix layer during production contains at least 40%-wt. of the readily volatile ingredient.

6. Active substance-containing plaster according to one or more of the preceding claims, characterized in that all matrix layers have an identical composition with regard to all the non-volatile ingredients and that after the migration of the readily volatile ingredient has terminated a matrix is obtained having a uniform, monolithic composition.

7. The active substance-containing plaster according to one or more of the preceding claims, characterized in that the first matrix layer, which contains the readily volatile ingredient, during production has a lesser thickness than the other matrix layers.

8. The active substance-containing plaster according to one or more of the preceding claims, characterized in that the first matrix layer, which contains the readily volatile active ingredient, during production has a layer thickness of between 2 and 100 µm, preferably 10 to 30 µm.

9. The active substance-containing plaster according to one or more of the preceding claims, characterized in that the readily volatile ingredient is a mixture of readily solvent substances.

10. The active substance-containing plaster according to one or more of the preceding claims, characterized in that the active substance contained therein is acetylsalicylic acid or one of the pharmaceutically acceptable salts thereof.

11. The active substance-containing plaster according to claim 9 or 10, characterized in that the main component of the said mixture of readily volatile substances is limonene.

12. A process for the production of active substance-containing plasters according to one or more of the preceding claims, characterized in that the layer containing the the readily volatile ingredient is evenly applied to the removable protective layer, and that after a laminate consisting of a second matrix layer and the backing layer has been laminated thereon, a pharmaceutically active plaster is obtained when the migration has terminated.

13. The process for the production of an active substance-containing plaster according to claim 12, characterized in that the composite obtained after lamination is subjected to a heat treatment to accelerate the migration.

## Revendications

1. Emplâtre contenant un principe actif muni d'un mécanisme de fixation de type auto-adhésif, contenant au moins un constituant très volatil, constitué par une couche dorsale essentiellement imperméable au principe actif, par au moins deux couches matricielles contenant un principe actif et par une couche de protection pelliculable, la couche matricielle contenant le principe actif très volatil étant contrecollée sur un composite ayant fait l'objet d'une préparation séparée et constitué par une ou plusieurs couches matricielles supplémentaires exemptes de principe actif, caractérisé en ce que la première couche matricielle contenant un principe actif est constituée par une solution dispersée au niveau moléculaire manifestant une aptitude à l'enduction de la matière de base de la matrice, dans le constituant très volatil utilisé à titre de solvant exclusif, dans lequel le composite obtenu après le contrecollage forme, après migration du constituant très volatil dans le composite, une matrice contenant un principe actif manifestant globalement une stabilité au cisaillement.

2. Emplâtre contenant un principe actif selon la revendication 1, caractérisé en ce que le constituant très volatil est un constituant pharmaceutiquement actif.

3. Emplâtre contenant un principe actif selon la revendication 1, caractérisé en ce que le constituant très volatil présente des propriétés favorisant la perméation à travers la peau.

4. Emplâtre contenant un principe actif selon une ou plusieurs des revendications précédentes, caractérisé en ce que la couche tournée vers la peau présente des propriétés auto-adhésives.

5. Emplâtre contenant un principe actif selon une ou plusieurs des revendications précédentes, caractérisé en ce que la première couche matricielle contient, lors de sa préparation, le constituant très volatil au moins à concurrence de 40% en poids.

6. Emplâtre contenant un principe actif selon une ou plusieurs des revendications précédentes, caractérisé en ce que toutes les couches matricielles présentent une composition identique en ce qui concerne tous les constituants non volatils et, au terme de la migration du constituant très volatil, on obtient une matrice monolithique et homogène quant à sa composition.

7. Emplâtre contenant un principe actif selon une ou plusieurs des revendications précédentes, caractérisé en ce que la première couche matricielle, qui contient le constituant très volatil, présente, lors de sa préparation, une épaisseur inférieure à celle des autres couches matricielles.

8. Emplâtre contenant un principe actif selon une ou plusieurs des revendications précédentes, caractérisé en ce que la première couche matricielle, qui contient le constituant très volatil, présente, lors de sa préparation, une épaisseur de couche entre 2 et 100 µm, de préférence de 10 à 30 µm.

9. Emplâtre contenant un principe actif selon une ou plusieurs des revendications précédentes, caractérisé en ce que le constituant très volatil est un mélange de substances très volatiles.

10. Emplâtre contenant un principe actif selon une ou plusieurs des revendications précédentes, caractérisé en ce que le principe actif qu'il contient représente l'acide acétylsalicylique ou un de ses sels pharmaceutiquement acceptables.

11. Emplâtre contenant un principe actif selon la revendication 9 ou 10, caractérisé en ce que la fraction principale du mélange mentionné de substances très volatiles représente le limonène.

12. Procédé pour la préparation d'emplâtres contenant un principe actif selon une ou plusieurs des revendications précédentes, caractérisé en ce qu'on applique la couche contenant le constituant très volatil de manière uniforme sur la couche de protection pelliculable et en ce que, après le contrecollage d'un stratifié constitué par une seconde couche matricielle et par la couche dorsale, au terme de la migration, on obtient un emplâtre pharmaceutiquement actif.

13. Procédé pour la préparation d'emplâtres contenant un principe actif selon la revendication 12, caractérisé en ce que le composite obtenu après le contrecollage est soumis à un traitement thermique pour accélérer la migration.
